# FASCICULE DE BREVET EUROPEEN

(11) **EP 2 308 814 B1**
(45) Date de publication et mention de la délivrance du brevet: **25.03.2015**
(21) Numéro de dépôt: 10290500.7
(22) Date de dépôt: 21.09.2010
(51) Int. Cl.: C07C 2/10, C10G 50/00, B01J 37/03, B01J 21/12, B01J 35/10, C07C 11/02

(54) **Procede d'oligomerisation d'une charge hydrocarbonee olefinique utilisant un catalyseur a base d'une silice-alumine mésoporeuse/macroporeuse**
Verfahren zur Oligomerisierung von Olefinen unter Verwendung von auf meso-/makroporöse Siliciumdioxid-Aluminiumdioxid basierter Katalysator
Oligomerization process of olefins using a mesoporous/macroporous silica-alumina catalyst

(30) Priorité: 08.10.2009 FR 0904818
(43) Date de publication de la demande: 13.04.2011
(73) Titulaire: IFP Energies nouvelles, 92852 Rueil-Malmaison Cedex (FR)
(72) Inventeur: Cabiac, Amandine, 69007 Lyon (FR); Chaumonnot, Alexandra, 69008 Lyon (FR)

(56) Documents cités:
- EP-A1- 1 616 846
- FR-A1- 2 887 556

## Description

### Domaine de l'invention

La présente invention se rapporte à tout procédé d'oligomérisation des oléfines permettant la production de carburants, par exemple la production d'essence et/ou de kérosène et/ou de gazole, à partir de charges oléfiniques légères contenant entre 2 et 10 atomes de carbone par molécule utilisant un catalyseur d'oligomérisation qui comprend au moins une silice-alumine présentant une distribution poreuse déterminée lorsqu'elle est mise en forme, la teneur massique en silice dudit catalyseur étant comprise entre 5 et 95% poids.

### Art Antérieur

Les procédés d'oligomérisation des oléfines légères destinés à la production d'oléfines de plus haut poids moléculaire sont largement utilisés dans le domaine du raffinage et de la pétrochimie, dans le but de valoriser les oléfines légères en bases pour carburants de type essence, kérosène ou gazole, ou bien en solvants. Les réactions d'oligomérisation sont conduites en présence d'un catalyseur, le plus souvent un catalyseur solide. Les oléfines se combinent en dimères, trimères, tétramères, etc., le degré de polymérisation des oléfines dépendant du type de catalyseur utilisé et des conditions opératoires de température et de pression imposées. L'avantage du procédé d'oligomérisation, par rapport à d'autres procédés dans le domaine du raffinage et de la pétrochimie conduisant à la même gamme de produits et bien connus de l'Homme du métier, réside dans le fait que les produits ainsi obtenus sont exempts de soufre et contiennent très peu de composés aromatiques. Les catalyseurs d'oligomérisation solides souvent cités dans la littérature sont des catalyseurs acides dont les exemples majeurs dans le domaine de l'oligomérisation d'oléfines légères sont des catalyseurs de type acide phosphorique imprégné sur support solide (par exemple US 2.913.506 et US 3.661.801), des silice-alumines (par exemple les brevets US 4.197.185, US 4.544.791 et EP 0.463.673), des zéolithes (par exemple les brevets US 4.642.404 et US 5.284.989) et, dans une moindre mesure, des hétéropolyanions (par exemple le brevet IN 170.903).

Les catalyseurs de type acide phosphorique imprégné sur support solide (SPA) présentent une bonne activité en oligomérisation ainsi qu'un rendement élevé en produits valorisables dans la coupe essence. Ces catalyseurs sont cependant difficiles à manipuler, en particulier au moment du déchargement de l'unité associée au procédé, du fait de leur tendance à prendre en masse en présence d'oléfines. De plus, lesdits catalyseurs de type acide phosphorique imprégné sur support solide se dégradent au cours de la réaction et ne sont pas régénérables. Les zéolithes sont des matériaux acides, actifs pour la réaction d'oligomérisation des oléfines légères du fait de la nature des sites engagés. Ces catalyseurs sont donc utilisés pour de telles applications. Un choix approprié du catalyseur zéolithique permet, via une sélectivité géométrique adaptée, la production accrue d'oligomères moins ramifiés que lors de l'emploi d'un catalyseur amorphe. Un choix judicieux du type de zéolithe comme catalyseur d'oligomérisation permet donc de moduler la sélectivité de la réaction et peut donc conduire à des oligomères possédant un taux de branchement inférieur à celui d'oligomères issus de réactions catalysées par des catalyseurs n'imposant aucune sélectivité de forme. Ce gain de sélectivité est favorable dans un contexte de production de gazole de bonne qualité, c'est-à-dire d'indice de cétane élevé, mais peu favorable par exemple à la production d'essence ayant un bon indice d'octane.
Les catalyseurs de type hétéropolyanions sont utilisés pour la réaction d'oligomérisation d'oléfines légères. Ces catalyseurs ne sont pas stables thermiquement et conduisent donc à des conversions faibles et des oligomères de degré de polymérisation limité du fait de la température de travail restreinte.
Le terme générique silice-alumine recouvre une large gamme de catalyseurs aluminosilicates amorphes présentant des propriétés texturales et physicochimiques adaptées à la réaction d'oligomérisation. Les propriétés de texture et d'acidité du matériau, dictées par le mode de préparation du catalyseur et bien connues de l'Homme du métier, conditionnent l'activité et la sélectivité du catalyseur. Il est connu que les catalyseurs à base de silice-alumine amorphe possédant un large volume poreux imposent moins de contraintes géométriques que leurs homologues zéolithiques et sont donc des candidats intéressants pour la production d'essence et/ou de kérosène de bonne qualité au travers de la réaction d'oligomérisation d'oléfines légères. Par exemple, les catalyseurs divulgués dans le brevet EP 1 616 846 ont un volume macroporeux réduit et dans le brevet EP 0.463.673 pour l'oligomérisation du propylène en produits valorisables dans le pool essence et/ou kérosène, sont des silice-alumines amorphes caractérisées par des surfaces spécifiques importantes, entre 500 et 1000 m²/g.
Une manière d'évaluer les performances d'un catalyseur d'oligomérisation consiste à estimer la sélectivité dudit catalyseur envers les produits de la réaction recherchés, à savoir des oligomères présentant une température d'ébullition inférieure à 225°C.
La sélectivité massique d'un catalyseur envers un produit P dans des conditions opératoires données est définie comme le rapport de la masse du produit P sur la somme des masses des produits de la réaction. La sélectivité envers le produit P est d'autant plus importante que les réactions secondaires, définies comme des réactions conduisant à la formation de produits différents du produit recherché, sont minimisées. Dans le cas de l'oligomérisation d'oléfines légères, les réactions d'oligomérisation successives ou les réactions d'oligomérisation non contrôlées conduisent à la production de produits possédant une masse moléculaire supérieure à la masse moléculaire des produits recherchés. D'autre part, les réactions de craquage conduisent à la production de produits possédant une masse moléculaire inférieure à la masse moléculaire des produits recherchés. Ces deux types de réactions sont donc à minimiser pour permettre d'améliorer la sélectivité envers un produit ou une famille de produits. Un des moyens permettant de minimiser ces réactions secondaires est de limiter les problèmes de diffusion au sein du lit catalytique.

### Résumé et intérêt de l'invention

La présente invention a pour objet un procédé d'oligomérisation d'une charge hydrocarbonée oléfinique consistant en la mise en contact de ladite charge avec au moins un catalyseur comprenant au moins une silice-alumine, la teneur massique en silice dudit catalyseur étant comprise entre 5 et 95% poids et la porosité de ladite silice-alumine mise en forme étant telle que :
i. le volume V1 des mésopores ayant un diamètre compris entre 4 et 15 nm représente de 30-80% du volume poreux total mesuré par intrusion au porosimètre à mercure,
ii. le volume V2 des macropores ayant un diamètre supérieur à 50 nm représente de 23 à 80% du volume poreux total mesuré par intrusion au porosimètre à mercure.

Un tel catalyseur est déjà connu de FR 2 887 556 mais pour une réaction d'hydroisomérisation/ hydrocraquage.

De manière préférée, le catalyseur d'oligomérisation est constitué intégralement de ladite silice-alumine et se présente sous forme d'extrudés.

Il a été découvert, de manière surprenante, qu'un catalyseur comprenant au moins une silice-alumine présentant une distribution poreuse déterminée lorsqu'elle est mise en forme, en particulier un volume macroporeux élevé, c'est-à-dire représentant de 23 à 80% du volume poreux total, conduit à des performances catalytiques améliorées en termes de sélectivité envers les produits désirés lorsqu'il est mis en oeuvre dans un procédé d'oligomérisation d'une charge hydrocarbonée oléfinique contenant des molécules oléfiniques ayant de 2 à 10 atomes de carbone par molécule, ledit procédé permettant la production de carburant, par exemple la production d'essence et/ou de kérosène et/ou de gazole. En effet, les propriétés physicochimiques particulières couplées à des propriétés texturales adaptées, en particulier les propriétés liées à la macroporosité, du catalyseur d'oligomérisation utilisé dans le procédé de ladite invention conduisent à une diminution des réactions secondaires décrites ci-dessus et donc à une amélioration de la sélectivité envers les produits recherchés lors de la mise en oeuvre dudit catalyseur dans un procédé d'oligomérisation d'une charge hydrocarbonée oléfinique contenant des molécules oléfiniques ayant de 2 à 10 atomes de carbone par molécule. Ainsi, l'amélioration de la diffusion intraparticulaire des réactifs et des produits au sein d'au moins la silice-alumine présente dans le catalyseur utilisé dans le procédé d'oligomérisation de l'invention se traduit par une meilleure sélectivité envers les oligomères recherchés, lesquels présentent une température d'ébullition généralement comprise entre 50 et 225°C. Plus précisément, le catalyseur d'oligomérisation mis en oeuvre dans le procédé de l'invention est plus sélectif non seulement envers les produits ayant une température d'ébullition inférieure à 155°C, correspondant aux produits incorporables dans une coupe essence, mais également envers les produits ayant une température d'ébullition comprise entre 155 et 225°C, correspondant aux produits incorporables dans une coupe kérosène. Le catalyseur d'oligomérisation mis en oeuvre dans le procédé selon l'invention favorise la production d'oligomères pouvant être facilement intégrés dans une coupe essence et/ou kérosène et/ou gazole au détriment de la production de produits plus lourds, non directement valorisables dans les coupes essence, kérosène et gazole désirées.

### Techniques de caractérisation

Le catalyseur à base d'au moins une silice-alumine, utilisé dans le procédé d'oligomérisation de l'invention, est caractérisé par plusieurs techniques d'analyses et notamment par Diffraction des Rayons X aux grands angles (DRX), par isotherme d'adsorption d'azote, par intrusion au porosimètre à mercure, par Microscopie Électronique à Transmission (MET) éventuellement couplée à une analyse par spectrométrie de rayons X à sélection d'énergie (EDX), par Rayonnement Magnétique Nucléaire du solide de l'atome d'aluminium (RMN MAS ²⁷Al), par spectroscopie infrarouge (IR) et par Fluorescence X (FX) ou Absorption Atomique (AA). La densité du catalyseur utilisé dans le procédé de l'invention est également évaluée.

La technique de Diffraction des Rayons X aux grands angles (valeurs de l'angle 2θ comprises entre 5° et 70°) permet de caractériser un solide cristallisé défini par la répétition d'un motif unitaire ou maille élémentaire à l'échelle moléculaire. Dans l'exposé qui suit, l'analyse des rayons X est réalisée sur poudre avec un diffractomètre opérant en réflexion et équipé d'un monochromateur arrière en utilisant la radiation du cuivre (longueur d'onde de 1,5406 Å). Les pics habituellement observés sur les diffractogrammes correspondant à une valeur donnée de l'angle 2θ sont associés aux distances inter-réticulaires d₍ₕₖₗ₎ caractéristiques de la (des) symétrie(s) structurale(s) du catalyseur ((hkl) étant les indices de Miller du réseau réciproque) par la relation de Bragg : 2 d ₍ₕₖₗ₎ * sin (θ) = n * λ. Cette indexation permet alors la détermination des paramètres de maille (abc) du réseau direct. Pour exemple et de manière avantageuse dans le cadre de l'invention, les 2 pics les plus intenses présents sur le diffractogramme du catalyseur d'oligomérisation utilisé pour la mise en oeuvre du procédé de l'invention sont situés à une position correspondant à un d compris entre 1,39 Å et 1,40 Å et un d compris entre 1,97 Å et 2,00 Å. Ces pics sont associés à la présence de l'alumine gamma dans la silice-alumine comprise dans le catalyseur d'oligomérisation. Par alumine gamma, on entend dans la suite du texte, entre autres et par exemple, une alumine comprise dans le groupe composé des alumines gamma cubique, gamma pseudo-cubique, gamma tétragonale, gamma mal ou peu cristallisée, gamma grande surface, gamma basse surface, gamma issue de grosse boehmite, gamma issue de boehmite cristallisée, gamma issue de boehmite peu ou mal cristallisée, gamma issue d'un mélange de boehmite cristallisée et d'un gel amorphe, gamma issue d'un gel amorphe, gamma en évolution vers delta. Pour les positions des pics de diffraction des alumines êta, delta et thêta, il est possible de se référer à l'article de B. C. Lippens, J. J. Steggerda, dans "Physical and Chemical Aspects of Adsorbents and Catalysts", E. G. Linsen (Ed.), Academic Press, London, 1970, 171. Pour le catalyseur utilisé dans le procédé selon l'invention, le diagramme de diffraction X met en évidence un pic large caractéristique de la présence de silice amorphe. Par ailleurs, dans l'ensemble du texte qui suit, la fraction aluminique du catalyseur d'oligomérisation peut contenir une fraction amorphe difficilement détectable par les techniques de DRX. Il sera donc sous-entendu par la suite que la fraction aluminique peut contenir une fraction amorphe ou mal cristallisée.

L'analyse isotherme d'adsorption d'azote correspondant à l'adsorption physique de molécules d'azote dans la porosité du catalyseur via une augmentation progressive de la pression à température constante renseigne sur les caractéristiques texturales (diamètre de pores, type de porosité, surface spécifique) particulières du catalyseur d'oligomérisation utilisé dans le procédé selon l'invention. En particulier, elle permet d'accéder à la surface spécifique et à la distribution mésoporeuse dudit catalyseur. On entend par surface spécifique, la surface spécifique B.E.T. (S_{BET} en m²/g) déterminée par adsorption d'azote conformément à la norme ASTM D 3663-78 établie à partir de la méthode BRUNAUER-EMMETT-TELLER décrite dans le périodique "The Journal of American Society", 1938, 60, 309. La distribution poreuse représentative d'une population de mésopores centrée dans une gamme de 1,5 nm à 50 nm est déterminée par le modèle Barrett-Joyner-Halenda (BJH). L'isotherme d'adsorption - désorption d'azote selon le modèle BJH est décrit dans le périodique *"*The Journal of American Society", 1951, 73, 373, écrit par E. P. Barrett, L. G. Joyner et P. P. Halenda. Dans l'exposé qui suit, le "volume adsorption azote du catalyseur" correspond au volume mesuré pour P/P₀ = 0,99, pression pour laquelle il est admis que l'azote a rempli tous les pores.

Dans l'exposé qui suit, le "volume mercure du catalyseur" correspond au volume mesuré par intrusion au porosimètre à mercure selon la norme ASTM D4284-83 à une pression maximale de 4000 bar, utilisant une tension de surface de 484 dyne/cm et un angle de contact pour le catalyseur d'oligomérisation comprenant au moins une silice-alumine amorphe de 140°. Le diamètre moyen mercure est défini comme étant un diamètre tel que tous les pores de taille inférieure à ce diamètre constituent 50% du volume poreux (V_{Hg}), dans un intervalle compris entre 3,6 nm et 100 nm. L'angle de mouillage a été pris égal à 140° en suivant les recommandations de l'ouvrage *"*Techniques de l'ingénieur, traité analyse et caractérisation", 1050, de J. Charpin et B. Rasneur. Afin d'obtenir une meilleure précision, la valeur du volume mercure en ml/g donnée dans le texte qui suit correspond à la valeur du volume mercure total en ml/g mesurée sur l'échantillon moins la valeur du volume mercure en ml/g mesurée sur le même échantillon pour une pression correspondant à 30 psi (environ 2 bar soit 0,2 MPa). Afin de mieux caractériser la distribution poreuse, on définit les critères de distribution poreuse suivants en mercure : le volume V1 qui est le volume correspondant aux pores présentant un diamètre compris dans la gamme de 4 nm à 15 nm, le volume V2 qui est le volume des macropores ayant un diamètre supérieur à 50 nm et le volume V3 qui est le volume des pores ayant un diamètre supérieur à 25 nm.

L'analyse par Microscopie Électronique par Transmission (MET) est une technique également largement utilisée pour caractériser le catalyseur d'oligomérisation comprenant au moins une silice-alumine utilisé dans le procédé d'oligomérisation selon l'invention. Celle-ci permet la formation d'une image du solide étudié, les contrastes observés étant caractéristiques de l'organisation structurale, de la texture, de la morphologie ou bien de la composition des particules observées, la résolution de la technique atteignant au maximum 0,2 nm. Pour cela un microscope électronique (du type Jeol 2010 ou Philips Tecnai20F éventuellement avec balayage) équipé d'un spectromètre de rayons X à sélection d'énergie (EDX) (par exemple un Tracor ou un Edax) est utilisé. Le détecteur EDX doit permettre la détection des éléments légers. L'association de ces deux outils, MET et EDX, permet de combiner l'imagerie et l'analyse chimique locale avec une bonne résolution spatiale. Pour ce type d'analyse, les échantillons sont finement broyés à sec dans un mortier. La poudre est ensuite incluse dans de la résine pour réaliser des coupes ultrafines d'épaisseur 70 nm environ. Ces coupes sont recueillies sur des grilles de cuivre recouvertes d'un film de carbone amorphe à trous servant de support. Elles sont ensuite introduites dans le microscope pour observation et analyse sous vide secondaire. En imagerie, les zones d'échantillon des zones de résine se distinguent aisément. Un certain nombre d'analyses sont ensuite effectuées, 10 au minimum, de préférence entre 15 et 30, sur différentes zones de l'échantillon. La taille du faisceau électronique pour l'analyse des zones (déterminant approximativement la taille des zones analysées) est de 50 nm de diamètre au maximum, de préférence de 20 nm, de manière encore plus préférée de 10, 5, 2 ou 1 nm de diamètre. En mode balayé, la zone analysée sera fonction de la taille de la zone balayée et non plus de la taille du faisceau généralement réduit. Le traitement semi-quantitatif des spectres X recueillis à l'aide du spectromètre EDX permet d'obtenir la concentration relative des éléments Al et Si (en % atomique) et le rapport atomique Si/Al pour chacune des zones analysées. La moyenne Si/Alₘ et l'écart type σ de cet ensemble de mesures peuvent alors être calculés.

Le catalyseur d'oligomérisation comprenant au moins une silice-alumine et utilisé dans le procédé de l'invention a été analysé par RMN MAS du solide de ²⁷Al sur un spectromètre de la firme Brüker de type MSL 400, en sonde 4 mm. La vitesse de rotation des échantillons est de l'ordre de 11 kHz. Potentiellement, la RMN de l'aluminium permet de distinguer trois types d'aluminium dont les déplacements chimiques sont reportés ci-après :
- entre 100 et 40 ppm, atomes d'aluminium de type tétracoordiné, notés Al_{IV},
- entre 40 et 20 ppm, atomes aluminium de type pentacoordiné, notés Al_{V},
- entre 20 et - 100 ppm, atomes aluminium de type hexacoordiné, notés Al_{VI}.
L'atome d'aluminium est un noyau quadripolaire. Dans certaines conditions d'analyse (champs de radiofréquence faible : 30 kHz, angle d'impulsion faible : π/2 et échantillon saturé en eau), la technique de RMN de rotation à l'angle magique (MAS) est une technique quantitative. La décomposition des spectres RMN MAS permet d'accéder directement à la quantité des différentes espèces. Le spectre est calé en déplacement chimique par rapport à une solution 1M de nitrate d'aluminium. Le signal d'aluminium est à zéro ppm. On a choisi d'intégrer les signaux entre 100 et 20 ppm pour les Al _{IV} et Al_{V}, ce qui correspond à l'aire 1, et entre 20 et -100 ppm pour Al_{VI}, ce qui correspond à l'aire 2. Dans l'exposé qui suit de l'invention, on entend par proportion des Al_{VI} octaédriques le rapport suivant : aire 2/(aire 1 + aire 2).

L'acidité du catalyseur d'oligomérisation est mesurée par spectroscopie infrarouge. Les spectres IR sont enregistrés sur un interféromètre Nicolet de type Nexus-670 sous une résolution de 4 cm⁻¹ avec une apodisation de type Happ-Gensel. L'échantillon (20 mg) est pressé sous la forme d'une pastille auto-supportée et placé dans une cellule d'analyse in situ (25 à 550°C, four déporté du faisceau IR, vide secondaire de 10⁻⁶ mbar). Le diamètre de la pastille est de 16 mm. L'échantillon est prétraité de la façon suivante afin d'éliminer l'eau physisorbée et de déshydroxyler partiellement la surface du catalyseur pour avoir une image représentative de l'acidité du catalyseur en fonctionnement :
- montée en température de 25 à 300°C en 3 heures,
- palier de 10 heures à 300°C,
- descente de température de 300 à 25°C en 3 heures.
La sonde basique (pyridine) est ensuite adsorbée à pression saturante à 25°C puis thermodésorbée selon les paliers suivants :
- 25°C pendant 2 heures sous vide secondaire,
- 100°C pendant 1 heure sous vide secondaire,
- 200°C pendant 1 heure sous vide secondaire,
- 300°C pendant 1 heure sous vide secondaire.
Un spectre est enregistré à 25°C à la fin du prétraitement et à chaque palier de désorption en mode transmission avec un temps d'accumulation de 100 s. Les spectres sont ramenés à isomasse (20 mg exactement). Le nombre de sites acides de Lewis est proportionnel à la surface du pic dont le maximum se situe vers 1450 cm⁻¹, tout épaulement étant inclu. Le nombre de sites acides de Brönsted est proportionnel à la surface du pic dont le maximum se situe vers 1545 cm⁻¹. Le rapport du nombre de sites acides de Brönsted/nombre de sites acides de Lewis (B/L) est estimé égal au rapport des surfaces des deux pics décrits ci-dessus. On utilise généralement la surface des pics à 25°C. Ce rapport B/L est, de manière générale, calculé à partir du spectre enregistré à 25°C après adsorption de la pyridine et du palier de 2 h sous vide secondaire.

La composition globale du catalyseur d'oligomérisation utilisé dans le procédé de l'invention, et en particulier la teneur en élément sodium, peut être déterminée par Fluorescence X (FX) sur ledit catalyseur à l'état pulvérulent ou par Absorption Atomique (AA) après attaque acide dudit catalyseur.

La densité de remplissage tassée (DRT) est mesurée, comme cela est décrit dans l'ouvrage "Applied Heterogeneous Catalysis" de J. F. Le Page, J. Cosyns, P. Courty, E. Freund, J. -P. Franck, Y. Jacquin, B. Juguin, C. Marcilly, G. Martino, J. Miquel, R. Montarnal, A. Sugier, H. Van Landeghem, Technip, Paris, 1987, chapitre 6.2.4, 167. Un cylindre gradué, de dimensions acceptables, est rempli par additions successives et, entre deux additions successives, le catalyseur est tassé en secouant le cylindre jusqu'à atteindre un volume constant. Cette mesure est généralement réalisée sur 1000 cm³ de catalyseur tassé dans un cylindre dont le ratio hauteur sur diamètre est proche de 5:1. Cette mesure peut être, de manière préférée, réalisée sur des appareils automatisés tels que Autotap® commercialisé par Quantachrome®.

### Description détaillée de l'invention

Dans la suite du texte, on entend par oligomérisation toute réaction d'addition d'une oléfine sur une autre oléfine.

La présente invention a pour objet un procédé d'oligomérisation d'une charge hydrocarbonée oléfinique consistant en la mise en contact de ladite charge avec au moins un catalyseur comprenant au moins une silice-alumine, la teneur massique en silice dudit catalyseur étant comprise entre 5 et 95% poids et la porosité de ladite silice-alumine mise en forme étant telle que :
i) le volume V1 des mésopores ayant un diamètre compris entre 4 et 15 nm représente de 30 à 80% du volume poreux total mesuré par intrusion au porosimètre à mercure,
ii) le volume V2 des macropores ayant un diamètre supérieur à 50 nm représente de 23 à 80% du volume poreux total mesuré par intrusion au porosimètre à mercure.

### Catalyseur utilisé dans le procédé d'oligomérisation selon l'invention

Le catalyseur utilisé dans le procédé d'oligomérisation selon la présente invention est un catalyseur non zéolitique comprenant au moins une silice-alumine, c'est-à-dire comprenant de la silice et de l'alumine.

Conformément à l'invention, le catalyseur d'oligomérisation présente une teneur massique en silice comprise entre 5 et 95% poids, de préférence entre 18 et 60% poids, de manière plus préférée entre 18 et 40% poids et de manière encore plus préférée entre 25 et 40% poids. En conséquence, le ratio molaire Si/Al du catalyseur d'oligomérisation utilisé dans le procédé selon l'invention est compris entre 0,044 et 17, de préférence entre 0,185 et 1,27, de manière plus préférée entre 0,185 et 0,56 et de manière encore plus préférée entre 0,28 et 0,56.

Conformément à l'invention, la silice-alumine mise en forme présente une distribution poreuse telle que :
i. le volume V1 des mésopores ayant un diamètre compris entre 4 et 15 nm représente de 30 à 80% du volume poreux total mesuré par intrusion au porosimètre à mercure,
ii. le volume V2 des macropores ayant un diamètre supérieur à 50 nm représente de 23 à 80% du volume poreux total, de préférence de 35 à 80% dudit volume poreux total mesuré par intrusion au porosimètre à mercure.
De manière avantageuse, la silice-alumine mise en forme présente un volume V3 des pores ayant un diamètre supérieur à 25 nm compris entre 20 et 80% du volume poreux total, de préférence entre 25% et 60% du volume poreux total et de manière encore plus préférée entre 30 et 50% du volume poreux total, mesuré par intrusion au porosimètre à mercure.
Ladite silice-alumine mise en forme, comprise dans le catalyseur utilisé dans le procédé d'oligomérisation selon l'invention, est une silice-alumine macroporeuse. La mise en forme de ladite silice-alumine est décrite plus loin dans la présente description. De manière préférée, ladite silice-alumine est mise en forme sous forme d'extrudés.
La silice-alumine mise en forme présente un volume poreux total mesuré par intrusion au porosimètre à mercure compris entre 0,45 et 0,96 ml/g. Le diamètre moyen des pores de la silice-alumine mise en forme, obtenu par intrusion au porosimètre à mercure, est compris dans une gamme de 2 à 15 nm, de préférence dans une gamme de 4 à 12 nm et de façon encore plus préférée dans une gamme de 5 à 12 nm. La silice-alumine mise en forme présente un volume poreux total mesuré par isotherme d'adsorption d'azote compris entre 0,45 et 0,96 ml/g.
Selon l'invention, la silice-alumine présente dans le catalyseur d'oligomérisation présente une teneur en impuretés cationiques préférentiellement inférieure à 0,1% poids, de manière plus préférée inférieure à 0,05% poids et de manière encore plus préférée inférieure à 0,025% poids. On entend par teneur en impuretés cationiques la teneur totale en alcalins, en particulier en sodium. Ladite silice-alumine présente une teneur en impuretés anioniques préférentiellement inférieure à 1% poids, de manière plus préférée inférieure à 0,5% poids et de manière encore plus préférée inférieure à 0,1 % poids. Les impuretés anioniques présentes dans ledit catalyseur d'oligomérisation sont notamment des halogénures, en particulier des chlorures, ainsi que des sulfates et des nitrates.
Selon l'invention, la silice-alumine mise en forme, présente dans le catalyseur d'oligomérisation, présente une surface spécifique BET comprise entre 100 et 550 m²/g, de préférence comprise entre 150 et 500 m²/g, de manière préférée comprise entre 150 et 350 m²/g et de manière encore plus préférée comprise entre 170 et 310 m²/g.

Selon l'invention, le diagramme de diffraction X du catalyseur d'oligomérisation contient avantageusement au moins les raies principales caractéristiques d'au moins une des alumines de transition comprises dans le groupe composé par les alumines alpha, rhô, khi, kappa, êta, gamma, thêta et delta et de manière préférée au moins les raies principales caractéristiques d'au moins une des alumines de transition comprise dans le groupe composé par l'alumine gamma, êta, thêta et delta, et de manière plus préférée au moins les raies principales caractéristiques de l'alumine gamma ou êta, et de manière encore plus préférée le diagramme contient les pics à un d compris entre 1,39 et 1,40 Å et à un d compris entre 1,97 Å et 2,00 Å. Le diffractogramme du catalyseur d'oligomérisation utilisé dans le procédé selon l'invention, obtenus par Diffraction aux Rayons X, correspond à un mélange de la silice et de l'alumine avec une certaine évolution entre l'alumine, préférentiellement l'alumine gamma, et la silice en fonction de la teneur en SiO₂ des échantillons. Plus précisément, l'analyse par diffraction des rayons X démontre l'augmentation du caractère amorphe aux rayons X qui s'accentue avec la teneur croissante en silice par comparaison avec l'alumine, préférentiellement l'alumine gamma.

La densité de remplissage tassée du catalyseur d'oligomérisation est supérieure à 0,40 g/cm³, de manière préférée supérieure à 0,45 g/cm³, de manière très préférée supérieure à 0,50 g/cm³.

La silice-alumine mise en forme, comprise dans le catalyseur utilisé dans le procédé selon l'invention, est de préférence une silice-alumine homogène à l'échelle du micromètre, c'est-à-dire qu'à l'échelle du micromètre, on ne distingue aucune séparation de phase entre la fraction aluminique et la fraction silicique présentes dans la silice-alumine. La silice-alumine mise en forme, présente dans le catalyseur utilisé dans le procédé selon l'invention, est de préférence une silice-alumine hétérogène à l'échelle du nanomètre, c'est-à-dire qu'à l'échelle du nanomètre, on distingue une séparation de phase entre la fraction aluminique et la fraction silicique présentes dans la silice-alumine. L'homogénéité à l'échelle du micromètre et l'hétérogénéité à l'échelle du nanomètre sont préférentiellement analysées par microscopie électronique à balayage (MEB).

Dans un mode de réalisation du catalyseur d'oligomérisation utilisé dans le procédé de l'invention, ledit catalyseur contient au moins deux zones silico-aluminiques, lesdites zones ayant des rapports molaires Si/Al inférieurs ou supérieurs au rapport molaire Si/Al global déterminé par fluorescence X. Ainsi un catalyseur ayant un rapport molaire Si/Al global égal à 0,5 comprend par exemple deux zones silico-aluminiques, l'une des zones ayant un rapport molaire Si/Al déterminé par MET inférieur à 0,5 et l'autre zone ayant un rapport molaire Si/Al déterminé par MET compris entre 0,5 et 17.

Dans un autre mode de réalisation du catalyseur d'oligomérisation utilisé dans le procédé de l'invention, ledit catalyseur contient une seule zone silico-aluminique, ladite zone ayant un rapport molaire Si/Al égal au rapport molaire Si/Al global déterminé par fluorescence X et supérieur à 0,044 et inférieur à 17.
Conformément à l'invention, ledit catalyseur utilisé dans le procédé d'oligomérisation de l'invention est préférentiellement constitué intégralement de ladite silice-alumine ; il est dépourvu de tout autre élément. La silice-alumine, mise en forme, présentant les caractéristiques en termes de volumes poreux V1, V2 et avantageusement V3 tels que définis plus haut dans la présente description constitue alors le catalyseur d'oligomérisation.

Toutefois, le catalyseur d'oligomérisation utilisé dans le procédé selon l'invention peut avantageusement comprendre un liant. Par exemple, ledit liant est choisi dans le groupe formé par la silice, l'alumine, les argiles, l'oxyde de titane, l'oxyde de bore et la zircone et tout mélange des liants précédemment cités. Les liants préférés sont la silice et l'alumine et de manière encore plus préférée l'alumine sous toutes ses formes connues de l'Homme du métier, par exemple l'alumine gamma. La teneur pondérale en liant dans le catalyseur est comprise entre 1 et 40% et de manière encore plus préférée entre 5% et 20%. Conformément à l'invention, la silice-alumine mise en forme avec un liant, préférentiellement avec une silice ou une alumine, constitue alors le catalyseur d'oligomérisation et présente les caractéristiques en terme de volumes poreux V1, V2 et avantageusement V3 tels que définis plus haut dans la présente description.

Les spectres RMN MAS du solide de ²⁷Al du catalyseur d'oligomérisation utilisé dans le procédé de l'invention montrent deux massifs de pics distincts. Un premier type d'aluminium dont le maximum résonne vers 10 ppm s'étend entre -100 et 20 ppm. La position du maximum suggère que ces espèces sont essentiellement de type Al_{VI} (octaédrique). Un deuxième type d'aluminium minoritaire dont le maximum résonne vers 60 ppm s'étend entre 20 et 110 ppm. Ce massif peut être décomposé en au moins deux espèces. L'espèce prédominante de ce massif correspondrait aux atomes d'Al_{IV} (tétraédrique). Pour le catalyseur d'oligomérisation utilisé dans le procédé d'oligomérisatioon de la présente invention, la proportion des Al_{VI} octaédriques est avantageusement supérieure à 50% molaire, de manière préférée supérieure à 60% molaire et de manière encore plus préférée supérieure à 70% molaire.

L'acidité du catalyseur d'oligomérisation utilisé dans le procédé selon l'invention est avantageusement mesurée par suivi IR de la thermodésorption de la pyridine. Généralement, le rapport B/L, tel que décrit plus haut dans la présente description, du catalyseur d'oligomérisation utilisé dans le procédé selon l'invention est compris entre 0,05 et 1, de manière préférée entre 0,05 et 0,7, de manière très préférée entre 0,05 et 0,5.

Le catalyseur d'oligomérisation utilisé dans le procédé de l'invention peut éventuellement contenir au moins un élément métallique choisi parmi les métaux des groupes IVB, VB, VIB et VIII et leurs mélanges. Parmi les métaux du groupe IVB, le titane, le zirconium et/ou l'hafnium peuvent être présents dans le catalyseur. Parmi les métaux du groupe VB, le vanadium, le niobium et/ou le tantale peuvent être présents dans le catalyseur. Parmi les métaux du groupe VIB, le chrome, le molybdène et/ou le tungstène peuvent être présents dans le catalyseur. Parmi les métaux du groupe VIII, les métaux appartenant à la première ligne des métaux du groupe VIII, à savoir le fer, le cobalt et le nickel, sont préférés. La teneur de ces métaux peut aller jusqu'à 10% poids du catalyseur final. Le catalyseur peut éventuellement contenir également du silicium comme élément dopant déposé sur la silice-alumine.

### Préparation du catalyseur utilisé dans le procédé d'oligomérisation selon l'invention

Tout procédé de synthèse de silice-alumine connu de l'Homme du métier conduisant à une silice-alumine homogène à l'échelle du micromètre et dans laquelle les impuretés cationiques peuvent être ramenées à moins de 0,1% poids et les impuretés anioniques peuvent être ramenées à moins de 1% poids et présentant une distribution poreuse en termes de volumes poreux V1, V2 et avantageusement V3 tels que définis plus haut dans la présente description convient pour préparer le catalyseur d'oligomérisation utilisé dans le procédé de l'invention. Un procédé de synthèse préféré consiste à procéder au mélange d'un composé de l'aluminium avec un composé de l'acide silicique dans un milieu aqueux, puis au séchage et à la calcination du produit obtenu. De manière préférée, on utilise des alcoolates d'aluminium comme précurseurs aluminiques et l'acide orthosilicique, avantageusement purifié sur des échangeurs d'ions, comme précurseur silicique. Le procédé de préparation de la silice-alumine présente dans le catalyseur d'oligomérisation comprend l'hydrolyse de l'alcoolate d'aluminium avec de l'eau, avantageusement purifiée sur des échangeurs d'ions, et l'ajout simultané ou subséquent de l'acide orthosilicique, avantageusement purifié sur des échangeurs d'ions. Plus préférentiellement, les alcoolates d'aluminium sont hydrolysés avec de l'eau purifiée sur des échangeurs d'ions, tandis que de l'acide orthosilicique purifié sur des échangeurs d'ions est de préférence ajouté simultanément en une quantité de 0,1 à 15 % en poids, de préférence de 0,1 à 10 % en poids, dans l'eau d'hydrolyse, ou bien le mélange alumine/eau obtenu après l'hydrolyse avec l'eau purifiée sur des échangeurs d'ions est mélangé avec la solution d'acide orthosilicique purifiée sur des échangeurs d'ions en une concentration de 0,1 à 15% en poids, de préférence de 0,1 à 10 % en poids. L'addition de l'acide orthosilicique au mélange alumine/eau peut par exemple se faire dans un réacteur à agitateur.

Suivant un mode de réalisation particulier du procédé de préparation de la silice-alumine, on ajoute conjointement avec la solution d'acide orthosilicique ou après l'addition de celle-ci une proportion mineure d'au moins un élément stabilisant choisi dans le groupe formé par la zircone et le titane. L'élément stabilisant est de préférence ajouté sous forme d'un sel soluble.

A l'issue de cette préparation, la silice-alumine est obtenue sous forme de poudre, laquelle est ensuite mise en forme comme décrit ci-après. L'obtention de ladite poudre peut être réalisée par précipitation/gélification et filtration, par atomisation d'une suspension et par toutes autres méthodes bien connues de l'Homme du métier.

### Mise en forme du catalyseur d'oligomérisation

Le catalyseur d'oligomérisation utilisé dans le procédé selon l'invention se présente sous la forme de sphères, de pastilles ou d'extrudés, préférentiellement d'extrudés. De manière très avantageuse, ledit catalyseur d'oligomérisation se présente sous la forme d'extrudés de diamètre compris entre 0,5 et 5 mm et plus particulièrement entre 0,7 et 2,5 mm. Les formes sont cylindriques (qui peuvent être creuses ou non), cylindriques torsadés, multilobées (2, 3, 4 ou 5 lobes par exemple). Les extrudés peuvent également être sous forme d'anneaux. Les formes cylindriques et multilobées sont utilisées de manière préférée, mais toute autre forme peut être utilisée.

Le catalyseur d'oligomérisation utilisé dans le procédé de l'invention est obtenu par mise en forme d'au moins ladite silice-alumine présente dans ledit catalyseur par toute technique connue de l'Homme du métier. La mise en forme peut être réalisée par exemple par extrusion, par dragéification, par pastillage, par séchage, par atomisation, par la méthode de la coagulation en goutte (oil-drop), par granulation au plateau tournant ou par toute autre méthode bien connue de l'Homme du métier. La mise en forme est réalisée en présence des différents constituants du catalyseur.

Plus précisément, quand le catalyseur se présente sous la forme d'extrudés, on peut ajouter ou retirer de l'eau pour ajuster la viscosité de la pâte à extruder. Cette étape peut être réalisée à tout stade de l'étape de malaxage de la silice-alumine. Pour ajuster la teneur en matière solide de la pâte à extruder afin de la rendre extrudable, on peut également ajouter un composé majoritairement solide et de préférence un oxyde ou un hydrate. On utilisera de manière préférée un hydrate et de manière encore plus préférée un hydrate d'aluminium. La perte au feu de cet hydrate est supérieure à 15%.

Le malaxage est préférentiellement réalisé en présence d'acide. La teneur en acide ajouté au malaxage avant la mise en forme est inférieure à 30%, de préférence comprise entre 0,5 et 20% poids de la masse anhydre en silice-alumine engagée dans la synthèse. L'extrusion peut être réalisée par n'importe quel outil conventionnel, disponible commercialement. La pâte issue du malaxage est extrudée à travers une filière, par exemple à l'aide d'un piston ou d'une monovis ou double vis d'extrusion. Cette étape d'extrusion peut être réalisée par toute méthode connue de l'Homme de métier. Les extrudés du catalyseur d'oligomérisation utilisé dans le procédé de l'invention ont généralement une résistance à l'écrasement d'au moins 70 N/cm et de manière préférée supérieure ou égale à 100 N/cm. De manière préférée, les extrudés sont constitués intégralement de silice-alumine et présentent les caractéristiques en termes de volumes poreux V1, V2 et avantageusement V3 tels que définis plus haut dans la présente description.

Par ailleurs, ledit catalyseur d'oligomérisation mis en oeuvre dans le procédé selon la présente invention peut avoir été traité ainsi qu'il est bien connu de l'Homme du métier par des additifs pour faciliter la mise en forme et/ou améliorer les propriétés mécaniques finales dudit catalyseur à base de silice-alumine. A titre d'exemple d'additifs, on peut citer notamment la cellulose, la carboxyméthylcellulose, la carboxyéthylcellulose, les gommes xanthaniques, des agents tensioactifs, des agents floculants comme les polyacrylamides, le noir de carbone, les amidons, l'acide stéarique, l'alcool polyacrylique, l'alcool polyvinylique, des biopolymères, le glucose, les polyéthylènes glycols, etc.

Le contrôle de la porosité caractéristique du catalyseur d'oligomérisation utilisé dans le procédé de l'invention est opéré partiellement lors de cette étape de mise en forme des particules de catalyseur.

### Séchage et calcination du catalyseur d'oligomérisation

Pour la préparation du catalyseur d'oligomérisation, une ou plusieurs étapes de séchage et une ou plusieurs étapes de calcination sont réalisées lors de la mise en oeuvre du procédé de préparation du catalyseur d'oligomérisation.

Le séchage est effectué par toute technique connue de l'Homme du métier. De manière préférée, le séchage est effectué à une température comprise entre 80 et 600°C, de préférence entre 300 et 600°C.

Pour obtenir le catalyseur utilisé dans le procédé d'oligomérisation de la présente invention, il est préférable de calciner de préférence en présence d'oxygène moléculaire, par exemple en effectuant un balayage d'air, à une température inférieure ou égale à 1100°C. Au moins une calcination peut être effectuée après l'une quelconque des étapes de la préparation. Ce traitement, par exemple, peut être effectué en lit traversé, en lit léché ou en atmosphère statique. Par exemple, le four utilisé peut être un four rotatif tournant ou être un four vertical à couches traversées radiales. Les conditions de calcination (température et durée) dépendent principalement de la température maximale d'utilisation du catalyseur, les conditions préférées de calcination se situant entre plus d'une heure à 200°C et moins d'une heure à 1100°C. La calcination peut être opérée en présence de vapeur d'eau. La calcination finale peut être éventuellement effectuée en présence d'une vapeur acide ou basique. Par exemple, la calcination peut être réalisée sous pression partielle d'ammoniaque.

Selon un mode de réalisation très préféré du procédé de préparation du catalyseur d'oligomérisation, on procède d'abord à l'obtention du solide constitué d'au moins ladite silice-alumine selon un procédé utilisant des alcoolates d'aluminium et de l'acide orthosilicique tel que décrit plus haut dans la présente description. Puis ledit solide est mis en forme sous forme d'extrudés de la manière décrite ci-dessus. Lesdits extrudés sont ensuite séchés et calcinés.

### Traitements post-synthèse mis en oeuvre sur le catalyseur mis en forme et calciné

Des traitements post-synthèse peuvent être effectués de manière à améliorer les propriétés du catalyseur, notamment son homogénéité à l'échelle du micromètre telle que définie précédemment.

Selon un mode de réalisation préféré, le traitement post-synthèse est un traitement hydrothermal. Le traitement hydrothermal est effectué par toute technique connue de l'Homme du métier. Par traitement hydrothermal, on entend la mise en contact du catalyseur d'oligomérisation avec de l'eau en phase vapeur ou en phase liquide. Par traitement hydrothermal, on peut entendre notamment mûrissement, steaming (traitement à la vapeur), autoclavage, calcination sous air humide, réhydratation. Sans que cela réduise la portée de l'invention, un tel traitement a pour effet de rendre mobile la composante silicique. Le mûrissement a lieu avantageusement avant ou après la mise en forme.

Selon un mode préféré de mise en oeuvre du traitement hydrothermal, on procède par steaming (traitement à la vapeur) dans un four en présence de vapeur d'eau. La température pendant le steaming (traitement à la vapeur) peut être comprise entre 600 et 1100°C et est de préférence supérieure à 700°C pendant une période de temps comprise entre 30 minutes et 3 heures. La teneur en vapeur d'eau est supérieure à 20 g d'eau par kg d'air sec et de préférence supérieure à 40 g d'eau par kg d'air sec et de manière préférée supérieure à 100 g d'eau par kg d'air sec. Un tel traitement peut, le cas échéant, remplacer totalement ou en partie le traitement de calcination.

Le catalyseur peut aussi être avantageusement soumis à un traitement hydrothermal en atmosphère confinée. On entend par traitement hydrothermal en atmosphère confinée un traitement du catalyseur par passage à l'autoclave en présence d'eau à une température supérieure à la température ambiante.

Au cours de ce traitement hydrothermal, on peut traiter de différentes manières le catalyseur comprenant ladite silice-alumine. Ainsi, on peut imprégner le catalyseur d'une solution acide, basique ou neutre, préalablement à son passage à l'autoclave, l'autoclavage du catalyseur étant fait soit en phase vapeur, soit en phase liquide, cette phase vapeur ou liquide de l'autoclave pouvant être acide, basique ou neutre. Cette imprégnation, préalable à l'autoclavage, peut être acide, basique ou neutre. Cette imprégnation, préalable à l'autoclavage peut être effectuée à sec ou par immersion du catalyseur dans une solution aqueuse acide, basique ou neutre. Par imprégnation à sec, on entend la mise en contact du catalyseur avec un volume de solution inférieur ou égal au volume poreux total de la silice-alumine traitée. De préférence, l'imprégnation est réalisée à sec.

L'autoclave est de préférence un autoclave à panier rotatif tel que celui défini dans la demande brevet EP-A-0 387 109.

La température pendant l'autoclavage peut être comprise entre 100 et 250°C pendant une période de temps comprise entre 30 minutes et 3 heures.

### Procédé d'oligomérisation selon l'invention

Le procédé selon l'invention est un procédé d'oligomérisation des oléfines permettant la production de carburant, par exemple la production d'essence et/ou de kérosène et/ou de gazole, à partir d'une charge hydrocarbonée oléfinique légère contenant entre 2 et 10 atomes de carbone par molécule, de préférence entre 2 et 8 atomes de carbone par molécule, et en particulier à partir d'une charge hydrocarbonée oléfinique légère contenant une forte proportion de propylène et/ou de butènes et/ou de pentènes utilisant un catalyseur d'oligomérisation comprenant au moins une silice-alumine, laquelle présente, après mise en forme, les caractéristiques en termes de volumes poreux V1, V2 et avantageusement V3 tels que définis plus haut dans la présente description.

La charge hydrocarbonée oléfinique employée dans le procédé d'oligomérisation selon l'invention contient de 20% à 100% en poids, et de préférence de 25% à 80% en poids d'oléfines. Les oléfines présentes dans la charge hydrocarbonée oléfinique peuvent provenir par exemple d'une unité de craquage catalytique et/ou d'une unité de vapocraquage et/ou d'une unité de déshydrogénation de paraffines et/ou d'une unité de déshydratation polymérisante de méthanol en eau et oléfines légères et/ou de toutes autres sources conduisant à la production d'oléfines légères.

### Charge mise en jeu dans le procédé d'oligomérisation selon l'invention

La charge hydrocarbonée oléfinique envoyée dans le réacteur d'oligomérisation utilisé pour la mise en oeuvre du procédé d'oligomérisation de l'invention, contenant ledit catalyseur à base d'au moins ladite silice-alumine, est de préférence débarrassée d'impuretés, telles que par exemple l'eau, les dérivés soufrés, les dérivés azotés basiques, avant d'être introduite dans le réacteur d'oligomérisation.

La charge hydrocarbonée oléfinique peut être une coupe C4 oléfinique, qui comprend habituellement à plus de 90% poids de l'isobutane, du n-butane, du 1-butène, des 2-butènes, de l'isobutène et éventuellement une petite quantité de butadiène. Le butadiène est généralement éliminé en amont de l'oligomérisation par un procédé d'hydrogénation sélective.

La charge hydrocarbonée oléfinique peut être également une coupe C3-C4 oléfinique. La composition de la coupe C3-C4 oléfinique est très variable selon sa provenance. Elle peut comprendre entre environ 20 et 50% poids de propylène et propane, entre environ 50 et 80% poids de l'isobutane, du n-butane, du 1-butène, des 2-butènes, de l'isobutène et éventuellement une petite quantité de butadiène. Le butadiène est généralement éliminé en amont de l'oligomérisation par un procédé d'hydrogénation sélective.

La charge hydrocarbonée oléfinique peut encore être une coupe C3 oléfinique. Elle comprend habituellement au moins 90% poids de propylène et de propane.

La charge hydrocarbonée oléfinique peut être aussi une coupe C5 oléfinique. La composition de la coupe C5 oléfinique est très variable selon sa provenance. Elle comprend avantageusement entre 30 et 80% poids de C5 oléfinique, entre 1 et 20% poids de C6 oléfinique et entre 1 et 20 % poids de C4 oléfinique.

La charge hydrocarbonée oléfinique peut être aussi une coupe contenant des oléfines avec plus de quatre atomes de carbone, notée coupe C4+. La composition de la coupe C4+ oléfinique est très variable selon sa provenance. Elle comprend avantageusement entre 30 et 80% poids de C4+ oléfinique.

Conformément à l'invention, l'exothermicité de la réaction d'oligomérisation peut être gérée par un recyclage d'au moins une partie de l'effluent non-converti, qui contient en particulier les paraffines qui n'ont pas été transformées lors de la réaction, vers le réacteur d'oligomérisation et/ou par dilution de la charge par un ajout de paraffines provenant d'une autre source, lesdites paraffines étant de même poids moléculaire et/ou plus lourdes que la charge oléfinique, lesdites paraffines étant aliphatiques ou cycliques, et/ou par recyclage d'une partie des oligomères formés.

Dans tous les cas de procédés conduisant à la formation d'essence et/ou de kérosène et/ou de gazole et/ou plus généralement d'une coupe oléfinique avec un point d'ébullition commençant à une température supérieure à 50°C, les coupes oléfiniques obtenues en sortie du procédé peuvent éventuellement être hydrogénées, partiellement ou totalement.

### Conditions opératoires du procédé d'oligomérisation selon l'invention

Ledit procédé d'oligomérisation est préférentiellement mis en oeuvre dans les conditions opératoires suivantes : la pression totale est comprise entre 0,1 et 20 MPa et préférentiellement entre 0,2 et 7 MPa, la température est comprise entre 30°C et 600°C et préférentiellement entre 40°C et 400°C, la vitesse spatiale horaire (VVH) est comprise entre 0,01 et 100 h⁻¹ et préférentiellement entre 0,05 et 20 h⁻¹.

Selon l'invention, le procédé d'oligomérisation correspond à une addition limitée à essentiellement 2 à 10 monomères ou molécules de base, lesdits monomères étant des oléfines.

### Modes de réalisation du procédé d'oligomérisation de l'invention

### Premier mode de réalisation : oligomérisation sélective

Selon ledit premier mode de réalisation, on met en contact une coupe C4 oléfinique avec le catalyseur comprenant ladite silice-alumine tel que décrit dans la présente invention de manière à limiter la conversion globale des n-butènes à moins de 10% poids, de manière préférée à moins de 5% poids, alors que plus de 90% poids de la quantité d'isobutène est convertie, de préférence plus de 95% poids. L'isobutène est converti à plus de 90% poids en dimères et trimères. Ensuite l'effluent d'oligomérisation est soumis à une distillation de telle sorte qu'une des fractions récupérées (effluent léger) contient à plus de 90% poids du butane, de l'isobutane et les butènes qui n'ont pas réagi lors de l'oligomérisation, une partie au moins de cette fraction alimentant ensuite par exemple une unité d'alkylation ou une unité d'hydratation, alors que l'autre fraction constituée des oligomères obtenus est utilisée comme base essence, éventuellement après hydrogénation partielle ou totale.

Le mode de réalisation du procédé d'oligomérisation décrit ci-dessus correspond au mode de réalisation dit d"oligomérisation sélective" dans lequel l'isobutène est majoritairement converti.

Conformément audit premier mode de réalisation du procédé d'oligomérisation de l'invention, la réaction d'oligomérisation est effectuée à une température comprise entre 30°C et 300°C, sous une pression comprise entre 0,1 et 20 MPa et le volume de charge hydrocarbonée oléfinique envoyé par volume de catalyseur et par heure est compris entre 0,05 et 5 h⁻¹. De préférence la température est comprise entre 40°C et 160°C et la pression entre 2 et 7 MPa, de façon à s'assurer que la réaction s'effectue en phase liquide, ou au moins en phase homogène (c'est-à-dire entièrement en phase liquide ou entièrement en phase gazeuse), et le volume de charge hydrocarbonée oléfinique envoyé par volume de catalyseur et par heure se situe de préférence entre 0,1 et 2,5 h⁻¹.

La technologie du réacteur d'oligomérisation peut être un lit fixe, un lit fluidisé ou un lit circulant. La technologie préférée est une mise en oeuvre en lit fixe.

De manière préférée, les oligomères ainsi obtenus sont réinjectés dans un réacteur d'oligomérisation supplémentaire contenant par exemple le catalyseur d'oligomérisation à base d'au moins ladite silice-alumine tel que précédemment décrit, de façon à augmenter la longueur de chaîne des oligomères et atteindre ainsi la coupe kérosène et/ou la coupe diesel, ou plus généralement une coupe oléfinique avec un point d'ébullition commençant à une température au moins supérieure à 150°C.

De manière avantageuse, l'effluent léger d'oligomérisation, c'est-à-dire la coupe C4, peut être introduit dans un réacteur d'hydroisomérisation visant à hydroisomériser une partie du 1-butène non-converti en 2-butène, de façon à se rapprocher de l'équilibre thermodynamique. Les autres constituants de l'effluent ne sont alors pas convertis de façon significative durant l'étape d'hydroisomérisation. La conversion du 1-butène en 2-butène est très utile si la fraction C4 ainsi obtenue en sortie du réacteur d'hydroisomérisation peut ensuite être introduite dans un réacteur d'alkylation aliphatique sur acide fluorhydrique, les produits obtenus par alkylation du 2-butène avec l'isobutane ayant un meilleur indice d'octane que l'alkylat obtenu à partir du 1-butène.

Étant donné la forte exothermicité de la réaction d'oligomérisation, la quantité d'isobutène dans la charge hydrocarbonée alimentant le réacteur d'oligomérisation est de préférence inférieure à 35% poids, de manière encore plus préférée inférieure à 15% poids, ladite quantité étant éventuellement obtenue en diluant la charge, par exemple avec du butane ou de l'isobutane ou du raffinat de l'unité d'oligomérisation.

### Deuxième mode de réalisation

Selon ledit deuxième mode de réalisation, on met en contact une coupe C4 oléfinique ou une coupe C3-C4 oléfinique avec le catalyseur d'oligomérisation comprenant au moins ladite silice-alumine tel que décrit précédemment dans la présente invention de manière à ce qu'une partie des butènes contenus dans la charge hydrocarbonée soit convertie en dimères ou trimères, utilisés ensuite comme base essence. Conformément audit deuxième mode de réalisation du procédé de l'invention, moins de 80% poids des butènes sont convertis et au moins 80% poids, de préférence au moins 90% poids de l'isobutène sont convertis. Ce procédé permet de produire une quantité maximale d'essence tout en minimisant les quantités de kérosène et de diesel formées.

Dans le réacteur d'oligomérisation utilisé pour la mise en oeuvre dudit deuxième mode de réalisation, la température se situe entre 40°C et 250°C, de préférence entre 50°C et 200°C, et la pression se situe entre 0,1 et 10 MPa, de préférence entre 0,1 et 6 MPa, et la quantité de charge hydrocarbonée envoyée par volume de catalyseur et par heure se situe entre 0,05 et 5 h⁻¹, de préférence entre 0,1 et 2,5 h⁻¹. La technologie du réacteur peut être un lit fixe, un lit fluidisé ou un lit circulant. La technologie préférée met en oeuvre un lit fixe.

Une variante dudit deuxième mode de réalisation du procédé de l'invention consiste à utiliser comme charge une charge oléfinique dans laquelle l'isobutène a été au préalable éliminé en partie ou totalement, par exemple en utilisant en amont de l'unité d'oligomérisation une unité d'éthérification en faisant réagir sélectivement l'isobutène avec un alcool, par exemple le méthanol ou l'éthanol, sans convertir le n-butène, ou bien en utilisant en amont de l'unité d'oligomérisation une unité d'oligomérisation sélective telle que celle décrite plus haut dans ledit premier mode de réalisation. Les oligomères produits présentent alors moins de branchements que ceux obtenus par traitement de la coupe complète incluant l'isobutène.

### Troisième mode de réalisation

Un troisième mode de réalisation du procédé selon l'invention consiste à soumettre une coupe C4 oléfinique, contenant éventuellement des traces de propylène, à une oligomérisation de telle sorte que la majeure partie des butènes contenus dans la charge soit convertie en dimères ou trimères, utilisés ensuite comme base essence. Conformément audit troisième mode de réalisation du procédé de l'invention, au moins 90% poids des 1-butène, au moins 80% poids des 2-butènes, au moins 97% poids de l'isobutène et au moins 80% poids du propylène sont convertis. Ledit troisième mode de réalisation du procédé de l'invention permet de produire une quantité maximale d'essence sans fabriquer de kérosène ou de diesel.

La coupe C4 oléfinique comprend habituellement de l'isobutane, du n-butane, du 1-butène, du 2-butène, de l'isobutène et éventuellement une petite quantité de butadiène. Le butadiène est généralement éliminé en amont de l'oligomérisation par un procédé d'hydrogénation sélective, pour éviter des réactions de polymérisation qui rendraient le catalyseur inactif.

Ledit procédé mis en oeuvre conformément audit troisième mode de réalisation comprend les étapes suivantes :
- une première étape d'oligomérisation : on traite une coupe C4 oléfinique, dans un premier réacteur d'oligomérisation dans lequel la conversion globale des n-butènes contenus dans la charge est inférieure à 45% poids et la conversion de l'isobutène est supérieure à 80% poids, de préférence supérieure à 85% poids, les oligomères obtenus étant des dimères et trimères à plus de 80% poids,
- on envoie l'effluent de la première étape d'oligomérisation dans une colonne de fractionnement de façon à récupérer une première fraction contenant l'isobutène et les n-butènes non-convertis et une deuxième fraction consistant à 90% poids en dimères et trimères de la réaction d'oligomérisation,
- une deuxième étape d'oligomérisation : ladite première fraction récupérée est introduite dans un deuxième réacteur d'oligomérisation dans lequel les oléfines sont converties en grande partie en dimères et trimères, c'est-à-dire qu'au moins 50% poids des n-butènes sont convertis, de préférence au moins 75% poids du 1-butène et au moins 55% poids du 2-butène sont convertis et,
- on envoie l'effluent de la deuxième étape d'oligomérisation dans la colonne de fractionnement associée au premier réacteur d'oligomérisation ou dans une deuxième colonne pour séparer l'essence ou le kérosène ou le diesel des composés en C4 non-convertis.
Dans les réacteurs d'oligomérisation, la température se situe entre 40°C et 250°C, de préférence entre 45°C et 200°C, et la pression se situe entre 0,1 et 10 MPa, de préférence entre 0,1 et 6 MPa, et la quantité de charge hydrocarbonée envoyée par volume de catalyseur et par heure se situe entre 0,05 et 5 h⁻¹, de préférence entre 0,1 et 2,5 h⁻¹. La technologie du réacteur peut être un lit fixe, un lit fluidisé ou un lit circulant. De préférence, la technologie est un lit fixe.

De préférence, dans le deuxième réacteur d'oligomérisation, les conditions opératoires sont plus sévères que dans le premier réacteur.

Ledit troisième mode de réalisation du procédé de l'invention peut être appliqué à une charge C3-C4 oléfinique.

### Quatrième mode de réalisation

Selon ledit quatrième mode de réalisation, on met une coupe C4 oléfinique ou une coupe C3-C4 oléfinique en contact avec le catalyseur d'oligomérisation comprenant ladite silice-alumine tel que décrit dans la présente invention de telle sorte que la majeure partie des butènes contenus dans la charge soit convertie, de façon à former une base essence et une base kérosène. Conformément audit quatrième mode de réalisation du procédé de l'invention, au moins 90% poids des 1-butène, au moins 80% poids des 2-butènes et au moins 97% poids de l'isobutène sont convertis. La coupe C4 oléfinique comprend habituellement essentiellement de l'isobutane, du n-butane, du 1-butène, du 2-butène, de l'isobutène et éventuellement une petite quantité de butadiène. La coupe C3-C4 oléfinique comprend en plus du propane et du propylène dans les proportions données plus haut dans la présente description.

Dans le réacteur d'oligomérisation, la température se situe entre 60°C et 250°C, de préférence entre 100°C et 200°C, et la pression se situe entre 0,1 et 10 MPa, de préférence entre 0,1 et 6 MPa, et la quantité de charge hydrocarbonée envoyée par volume de catalyseur et par heure se situe entre 0,05 et 5 h⁻¹, de préférence entre 1 et 2,5 h⁻¹. La technologie du réacteur peut être un lit fixe, un lit fluidisé ou un lit circulant. De préférence la technologie est un lit fixe.

### Cinquième mode de réalisation

Selon ledit cinquième mode de réalisation, on met une coupe C3 oléfinique en contact avec ledit catalyseur d'oligomérisation comprenant ladite silice-alumine tel que décrit dans la présente invention de telle sorte que la majeure partie du propylène contenu dans la charge soit convertie, c'est à dire qu'au moins 80% poids du propylène contenu dans la charge sont convertis, de façon à former une base essence et une base kérosène. La coupe C3 oléfinique comprend habituellement au moins 90% poids de propylène et de propane.

La réaction d'oligomérisation est effectuée à une température entre 30°C et 300°C, sous une pression entre environ 0,1 et 20 MPa et le volume de charge hydrocarbonée envoyé par volume de catalyseur et par heure se situe entre 0,05 et 5 h⁻¹. De préférence la température se situe entre 40°C et 160°C, la pression se situe entre 2 et 7 MPa, le volume de charge hydrocarbonée envoyé par volume de catalyseur et par heure se situe de préférence entre 0,1 et 2,5 h-1. La technologie du réacteur peut être un lit fixe, un lit fluidisé ou un lit circulant. La technologie préférée est une mise en oeuvre en lit fixe.

### Sixième mode de mode de réalisation

Selon ledit sixième mode de réalisation, on met une coupe oléfinique contenant des oléfines avec plus de quatre atomes de carbone, par exemple une coupe provenant d'un procédé FCC (craquage catalytique en lit fluidisé), en contact avec ledit catalyseur d'oligomérisation comprenant ladite silice-alumine tel que décrit dans la présente invention de telle sorte que la majeure partie des oléfines contenant au moins 4 atomes de carbone contenues dans la charge soit convertie, c'est à dire qu'au moins 70% poids des oléfines contenues dans la charge soient converties, de façon à former une base essence, une base kérosène ou une base diesel.

La réaction d'oligomérisation est effectuée à une température entre 30°C et 300°C, sous une pression entre environ 0,1 et 20 MPa et le volume de charge hydrocarbonée envoyé par volume de catalyseur et par heure se situe entre 0,05 et 5 h⁻¹. De préférence la température se situe entre 40°C et 160°C, et la pression entre 2 et 7 MPa, le volume de charge hydrocarbonée envoyé par volume de catalyseur et par heure se situe de préférence entre 0,1 et 2,5 h⁻¹. La technologie du réacteur peut être un lit fixe, un lit fluidisé ou un lit circulant. La technologie préférée est une mise en oeuvre en lit fixe. Le mode de transformation mettant en oeuvre un schéma de recycle peut être également utilisé.

Les exemples qui suivent illustrent la présente invention sans en limiter la portée.

### Exemple 1 : préparation d'un catalyseur C1 constitué d'une silice-alumine SA1 (conforme à l'invention).

Le catalyseur C1 est préparé par extrusion d'une silice alumine SA1 sans liant. Dans cet exemple, le catalyseur C1 se confond donc avec la silice-alumine SA1 mise en forme. Le catalyseur C1 a une composition chimique en poids de 71 % de Al₂O₃ et 29% de SiO₂.
On hydrolyse en marche continue dans un réacteur agité, à 90°C pendant 45 minutes, 45,0 g d'hexanolate d'aluminium au moyen de 45,5 g d'eau déminéralisée. On mélange ensuite la suspension aqueuse d'alumine qui se sépare des alcools avec de l'acide orthosilicique déminéralisé sur un échangeur d'ions en une quantité totale de 8,9% en poids. On sèche ensuite la suspension obtenue classiquement à l'aide d'un atomiseur de manière conventionnelle à une température de 300°C à 600°C et on obtient ainsi la silice-alumine SA1. La poudre ainsi préparée est mise en forme dans un bras en Z en présence de 3% poids d'acide nitrique par rapport au produit anhydre. L'extrusion est réalisée par passage de la pâte au travers d'une filière munie d'orifices de diamètre égal à 2,5 mm. Les extrudés du catalyseur C1 ainsi obtenus sont séchés à 150°C, puis calcinés à 550°C. Les rapports Si/Al mesurés par analyses FX et MET couplée EDX du catalyseur C1 sont respectivement de 0,35 et 0,33. Les teneurs en sodium et en chlore du catalyseur C1 sont respectivement de 0,01% et 0,02% en poids. Les extrudés sont cylindriques de diamètre égal à 1,6 mm. La surface spécifique du catalyseur C1 est de 307 m²/g. Le volume poreux total du catalyseur C1, mesuré par isotherme d'adsorption d'azote est égal à 0,59 ml/g. Son volume poreux total, mesuré par porosimétrie au mercure est égal à 0,78 ml/g. Le diamètre moyen des pores du catalyseur C1 déterminé à partir de la porosimétrie mercure est de 7,6 nm. Le volume V1 des mésopores avec un diamètre compris entre 4 et 15 nm est de 0,40 ml/g et ce volume représente environ 51% du volume poreux total. Le volume V2 des macropores du catalyseur, dont le diamètre est plus grand que 50 nm, est de 0,32 cm³/g et représente environ 41% du volume poreux total. Le volume V3 des pores du catalyseur, dont le diamètre est plus grand que 25 nm, est de 0,35 ml/g et représente environ 45% du volume poreux total. La DRT du catalyseur est de 0,50 g/cm³. Le rapport B/L a une valeur de l'ordre de 0,1.

### Exemple 2 : préparation et mise en forme d'un catalyseur C2 constitué d'une silice-alumine SA2 (non conforme à l'invention).

Le catalyseur C2 est préparé par extrusion d'une silice-alumine SA2 avec de la boehmite. La composition chimique en poids du catalyseur C2 est de 71 % de Al₂O₃ et 29% de SiO₂.
Une solution aqueuse de sulfate d'aluminium (6,1% poids en aluminium) et une solution aqueuse à base de silicate de sodium (10,2% poids en silicium) et de soude (9,7% en poids), maintenues à une température de 30°C, sont introduites dans un réacteur contenant 1,21 d'eau déminéralisée chauffée à 30°C. Le pH de l'ensemble de la solution lors de l'ajout dans le réacteur est maintenu à 8 grâce à une solution d'acide sulfurique. La suspension ainsi obtenue est filtrée. La silice-alumine SA2 résultante a une composition chimique en poids de 30% de Al₂O₃ et de 70% de SiO₂. Le gâteau de filtration de SA2 est récupéré puis malaxé dans un bras en Z en présence de 3% poids d'acide nitrique, par rapport au produit anhydre, et de boehmite de façon à atteindre la composition chimique en poids du catalyseur C2 de 71 % de Al₂O₃ et 29% de SiO₂. L'extrusion est réalisée par passage de la pâte au travers d'une filière munie d'orifices de diamètre égal à 2,5 mm. Les extrudés du catalyseur C2 ainsi obtenus sont séchés à 150°C, puis calcinés à 550°C.
La surface spécifique du catalyseur C2 est de 260 m²/g. Son volume poreux total, mesuré par isotherme d'adsorption d'azote est égal à 0,57 ml/g. Son volume poreux total, mesuré par porosimétrie au mercure est égal à 0,44 ml/g. Le diamètre moyen des pores du catalyseur C2 déterminé à partir de la porosimétrie mercure est de 7,7 nm. Le volume V1 des mésopores (diamètre compris entre 4 et 15 nm) du catalyseur C2 est de 0,40 ml/g et ce volume représente environ 83% du volume poreux total. Le volume V2 des macropores (diamètre supérieur à 50 nm) du catalyseur C2 est de 0,021 ml/g et représente environ moins de 5% du volume poreux total. Le volume V3 des pores (diamètre supérieur à 25 nm) du catalyseur C2 est de 0,07 ml/g et représente moins de 16% du volume poreux total. La DRT du catalyseur est de 0,61 g/cm³.

### Exemple 3 : évaluation catalytique des catalyseurs C1 et C2 dans un procédé d'oligomérisation d'oléfines légères (premier mode de réalisation).

Une coupe C4 oléfinique issue d'une unité de craquage catalytique est séchée sur un tamis moléculaire de type 13X pour éliminer les traces de soufre et d'eau. La composition de la charge à l'issue de ces traitements est rapportée dans le Tableau 1.

**Tableau 1 : composition de la charge d'oligomérisation d'oléfines légères.**

| Composés | Teneur (% pds.) |
|---|---|
| Isobutane | 30,7 |
| n-butane | 12,7 |
| isobutène | 15,6 |
| 1-butène | 9,4 |
| 2-butène | 31,6 |

Les catalyseurs C1 et C2 sont chargés dans un réacteur en lit fixe et testés pour la réaction d'oligomérisation de la charge décrite dans le Tableau 1. Le descriptif des conditions opératoires appliquées est donné dans le tableau 2. Les catalyseurs sont activés in situ avant la réaction d'oligomérisation sous N₂ à 250°C pendant 4 heures.

**Tableau 2 : conditions opératoires de la réaction d'oligomérisation.**

| | |
|---|---|
| Gamme de pression | 25-65 MPa |
| Gamme de température | 60°C-120°C |
| VVH | 0,85 h⁻¹ |

| | |
|---|---|
| VVH (h⁻¹) = [volume de catalyseur / débit volumique de charge] : 0,85 h⁻¹ | |

Les performances des catalyseurs sont évaluées en fonction de la sélectivité en produit recherché pour des conversions en oléfines équivalentes. La sélectivité de la coupe 155⁻ est définie comme le rapport de la masse de produits présentant une température d'ébullition inférieure à 155°C sur la masse globale de produits de la réaction. La coupe 155⁻ qui comprend des produits présentant une température d'ébullition inférieure à 155°C est une coupe essence. De la même manière, la sélectivité de la coupe 155-225 est définie comme la fraction massique de produits présentant une température d'ébullition dans la gamme 155°C - 225°C. La coupe 155-225 qui comprend des produits présentant une température d'ébullition dans la gamme 155°C - 225°C est une coupe kérosène. Les sélectivités massiques pour les coupes 155⁻ et 155-225 des catalyseurs C1 et C2 aux conditions opératoires du test sont résumées dans le tableau 3.

**Tableau 3 : performances des catalyseurs C1 et C2.**

| Catalyseur | Catalyseur C1 | | Catalyseur C2 | |
|---|---|---|---|---|
| Température de la réaction | 60°C | 110°C | 60°C | 110°C |
| Sélectivité de la coupe 155⁻ | 62,0 | 56,5 | 60,0 | 54,0 |
| Sélectivité de la coupe 155-225 | 32,2 | 33,3 | 33,7 | 34,6 |
| Sélectivité de la coupe 225⁻ | 94,2 | 89,8 | 93,7 | 88,6 |
| Sélectivité de la coupe 225⁺ | 5,8 | 10,2 | 6,3 | 11,4 |

A iso-conversion en oléfines, le catalyseur C1 formé d'une silice-alumine macroporeuse après mise en forme est plus sélectif que le catalyseur C2 comprenant une silice-alumine non macroporeuse après mise en forme : le catalyseur C1 favorise la production d'oligomères présentant une température d'ébullition inférieure à 225°C, au détriment de la production de produits plus lourds présentant une température d'ébullition supérieure à 225°C.

## Revendications

1. Procédé d'oligomérisation d'une charge hydrocarbonée oléfinique consistant en la mise en contact de ladite charge avec au moins un catalyseur comprenant au moins une silice-alumine, la teneur massique en silice dudit catalyseur étant comprise entre 5 et 95% poids et la porosité de ladite silice-alumine mise en forme étant telle que :
i. le volume V1 des mésopores ayant un diamètre compris entre 4 et 15 nm représente de 30-80% du volume poreux total mesuré par intrusion au porosimètre à mercure,
ii. le volume V2 des macropores ayant un diamètre supérieur à 50 nm représente de 23 à 80% du volume poreux total mesuré par intrusion au porosimètre à mercure.

2. Procédé d'oligomérisation selon la revendication 1 tel que ledit catalyseur présente une teneur massique en silice comprise entre 25 et 40% poids.

3. Procédé d'oligomérisation selon la revendication 1 ou la revendication 2 tel que ladite silice-alumine mise en forme présente une distribution poreuse telle que ledit volume V2 des macropores représente de 35 à 80% du volume poreux total mesuré par intrusion au porosimètre à mercure.

4. Procédé d'oligomérisation selon l'une des revendication 1 à 3 tel que le diamètre moyen des pores de la silice-alumine mise en forme, obtenu par intrusion au porosimètre à mercure, est compris dans une gamme de 2 à 15 nm.

5. Procédé d'oligomérisation selon l'une des revendications 1 à 4 tel que ladite silice-alumine mise en forme présente un volume V3 des pores ayant un diamètre supérieur à 25 nm compris entre 20 et 80% du volume poreux total mesuré par intrusion au porosimètre à mercure.

6. Procédé d'oligomérisation selon l'une des revendications 1 à 5 tel que ladite silice-alumine mise en forme présente une surface spécifique BET comprise entre 100 et 550 m²/g.

7. Procédé d'oligomérisation selon l'une des revendications 1 à 6 tel que ladite silice-alumine mise en forme est une silice-alumine homogène à l'échelle du micromètre.

8. Procédé d'oligomérisation selon l'une des revendications 1 à 7 tel que ledit catalyseur est constitué intégralement de ladite silice-alumine.

9. Procédé d'oligomérisation selon l'une des revendications 1 à 7 tel que ledit catalyseur comprend un liant.

10. Procédé d'oligomérisation selon l'une des revendications 1 à 9 tel que ladite charge hydrocarbonée oléfinique contient de 25 à 80% en poids d'oléfines.

11. Procédé d'oligomérisation selon l'une des revendications 1 à 10 tel que ladite charge hydrocarbonée oléfinique est une coupe C3 oléfinique comprenant au moins 90% poids de propylène et de propane.

12. Procédé d'oligomérisation selon l'une des revendications 1 à 10 tel que ladite charge hydrocarbonée oléfinique est une coupe C3-C4 oléfinique.

13. Procédé d'oligomérisation selon l'une des revendications 1 à 10 tel que ladite charge hydrocarbonée oléfinique est une coupe C4 oléfinique comprenant à plus de 90% poids de l'isobutane, du n-butane, du 1-butène, des 2-butènes, de l'isobutène.

14. Procédé d'oligomérisation selon l'une des revendications 1 à 10 tel que ladite charge hydrocarbonée oléfinique est une coupe C5 oléfinique.

## Patentansprüche

1. Verfahren zur Oligomerisierung einer olefinhaltigen Kohlenwasserstoffbeschickung, das darin besteht, die Beschickung mit mindestens einem Katalysator, umfassend mindestens ein Siliciumdioxid-Aluminiumoxid, in Kontakt zu bringen, wobei der Massegehalt an Siliciumdioxid des Katalysators im Bereich zwischen 5 und 95 Gew.-% liegt und die Porosität des geformten Siliciumdioxid-Aluminiumoxids derart ist, dass:
i. das Volumen V1 der Mesoporen mit einem Durchmesser zwischen 4 und 15 nm 30 bis 80 % des Gesamtporenvolumens, gemessen mittels Quecksilberintrusion-Porosimetrie, ausmacht,
ii. das Volumen V2 der Makroporen mit einem Durchmesser größer als 50 nm 23 bis 80 % des Gesamtporenvolumens, gemessen mittels Quecksilberintrusion-Porosimetrie, ausmacht.

2. Verfahren zur Oligomerisierung nach Anspruch 1, wobei der Katalysator einen Massegehalt an Siliciumdioxid zwischen 25 und 40 Gew.-% autweist.

3. Verfahren zur Oligomerisierung nach Anspruch 1 oder 2, wobei das geformte Siliciumdioxid-Aluminiumoxid eine derartige Porenverteilung aufweist, dass das Volumen V2 der Makroporen 35 bis 80 % des Gesamtporenvolumens, gemessen mittels Quecksilberintrusion-Porosimetrie, ausmacht.

4. Verfahren zur Oligomerisierung nach einem der Ansprüche 1 bis 3, wobei der mittlere Porendurchmesser des geformten Siliciumdioxid-Aluminiumoxids, erhalten mittels Quecksilberintrusion-Porosimetrie, im Bereich zwischen 2 bis 15 nm liegt.

5. Verfahren zur Oligomerisierung nach einem der Ansprüche 1 bis 4, wobei das geformte Siliciumdioxid-Aluminiumoxid ein Volumen V3 der Poren mit einem Durchmesser größer als 25 nm zwischen 20 und 80 % des Gesamtporenvolumens, gemessen mittels Quecksilberintrusion-Porosimetrie, aufweist.

6. Verfahren zur Oligomerisierung nach einem der Ansprüche 1 bis 5, wobei das geformte Siliciumdioxid-Aluminiumoxid eine spezifische BET-Oberfläche zwischen 100 und 550 m²/g aufweist.

7. Verfahren zur Oligomerisierung nach einem der Ansprüche 1 bis 6, wobei das geformte Siliciumdioxid-Aluminiumoxid ein im Mikrometerbereich homogenes Siliciumdioxid-Aluminiumoxid ist.

8. Verfahren zur Oligomerisierung nach einem der Ansprüche 1 bis 7, wobei der Katalysator vollständig aus dem Siliciumdioxid-Aluminiumoxid besteht.

9. Verfahren zur Oligomerisierung nach einem der Ansprüche 1 bis 7, wobei der Katalysator ein Bindemittel umfasst.

10. Verfahren zur Oligomerisierung nach einem der Ansprüche 1 bis 9, wobei die olefinhaltige Kohlenwasserstoffbeschickung 25 bis 80 Gew.-% Olefine enthält.

11. Verfahren zur Oligomerisierung nach einem der Ansprüche 1 bis 10, wobei die olefinhaltige Kohlenwasserstoffbeschickung ein olefinhaltiger C3-Schnitt ist, umfassend mindestens 90 Gew.-% Propylen und Propan.

12. Verfahren zur Oligomerisierung nach einem der Ansprüche 1 bis 10, wobei die olefinhaltige Kohlenwasserstoffbeschickung ein olefinhaltiger C3-C4-Schnitt ist.

13. Verfahren zur Oligomerisierung nach einem der Ansprüche 1 bis 10, wobei die olefinhaltige Kohlenwasserstoffbeschickung ein olefinhaltiger C4-Schnitt ist, umfassend mehr als 90 Gew.-% Isobutan, n-Butan, 1-Buten, 2-Butene, Isobuten.

14. Verfahren zur Oligomerisierung nach einem der Ansprüche 1 bis 10, wobei die olefinhaltige Kohlenwasserstoffbeschickung ein olefinhaltiger C5-Schnitt ist.

## Claims

1. Method of oligomerization of an olefinic hydrocarbon feed consisting of contacting said feed with at least one catalyst comprising at least one silica-alumina, the silica content by weight of said catalyst being between 5 and 95 wt.% and the porosity of said silica-alumina when formed being such that:
i) the volume V1 of mesopores having a diameter comprised between 4 and 15 nm represents 30-80% of the total pore volume measured with a mercury intrusion porosimeter,
ii) the volume V2 of macropores having a diameter greater than 50 nm represents from 23 to 80% of the total pore volume measured with a mercury intrusion porosimeter.

2. Method of oligomerization according to claim 1, **characterized in that** said catalyst has a silica content by weight between 25 and 40 wt.%.

3. Method of oligomerization according to claim 1 or claim 2, **characterized in that** said silica-alumina when formed has a pore distribution such that said volume V2 of macropores represents from 35 to 80% of the total pore volume measured with a mercury intrusion porosimeter.

4. Method of oligomerization according to one of claims 1 to 3, **characterized in that** the average diameter of the pores of the formed silica-alumina, obtained using the mercury intrusion porosimeter, is in a range from 2 to 15 nm.

5. Method of oligomerization according to one of claims 1 to 4, **characterized in that** said silica-alumina when formed has a volume V3 of pores having a diameter greater than 25 nm between 20 and 80% of the total pore volume measured with a mercury intrusion porosimeter.

6. Method of oligomerization according to one of claims 1 to 5, **characterized in that** said silica-alumina when formed has a BET specific surface between 100 and 550 m²/g.

7. Method of oligomerization according to one of claims 1 to 6, **characterized in that** said silica-alumina when formed is a silica-alumina that is homogeneous at the micrometre scale.

8. Method of oligomerization according to one of claims 1 to 7, **characterized in that** said catalyst is constituted entirely by said silica-alumina.

9. Method of oligomerization according to one of claims 1 to 7, **characterized in that** said catalyst contains a binder.

10. Method of oligomerization according to one of claims 1 to 9, **characterized in that** said olefinic hydrocarbon feed contains from 25 to 80 wt.% of olefins.

11. Method of oligomerization according to one of claims 1 to 10, **characterized in that** said olefinic hydrocarbon feed is an olefinic C3 cut comprising at least 90 wt.% of propylene and propane.

12. Method of oligomerization according to one of claims 1 to 10, **characterized in that** said olefinic hydrocarbon feed is an olefinic C3-C4 cut.

13. Method of oligomerization according to one of claims 1 to 10, **characterized in that** said olefinic hydrocarbon feed is an olefinic C4 cut comprising, to more than 90 wt.%, isobutane, n-butane, 1-butene, 2-butenes, isobutene.

14. Method of oligomerization according to one of claims 1 to 10, **characterized in that** said olefinic hydrocarbon feed is an olefinic C5 cut.
